# EUROPEAN PATENT SPECIFICATION

(11) **EP 1 181 025 B1**
(45) Date of publication and mention of the grant of the patent: **13.08.2003**
(21) Application number: 00940329.6
(22) Date of filing: 06.06.2000
(51) Int. Cl.: A61K 35/12, A61K 35/74, A61K 35/76, A61K 39/00, C12N 5/06, C12N 5/08, A61P 35/00, A61P 31/00, A61P 43/00

(54) **MOLECULAR COMPLEXES PRESENTING HIGH AFFINITY BINDING WITH RESPECT TO MONOCYTE DERIVED CELLS AND THEIR USES IN THERAPY**
MOLEKULÄRE KOMPLEXE MIT HOHER AFFINITÄT FÜR MONOZYT-DERIVIERTE ZELLEN UND DEREN THERAPEUTISCHE VERWENDUNG
COMPLEXES MOLECULAIRES A FORTE AFFINITE DE LIAISON AVEC DES CELLULES MONOCYTES ET LEURS APPLICATIONS THERAPEUTIQUES

(30) Priority: 09.06.1999 EP 99401385
(43) Date of publication of application: 27.02.2002
(73) Proprietor: I.D.M. IMMUNO-DESIGNED MOLECULES, 75011 Paris (FR)
(72) Inventor: BARTHOLEYNS, Jacques Manoir de la Vignole, F-49730 Turquant (FR); ROMET-LEMONNE, Jean-Loup, F-75003 Paris (FR)
(74) Representative: Grosset-Fournier, Chantal Catherine
(86) International application number: EP0005202
(87) International publication number: WO00076527

(56) References cited:
- WO-A-97/01760
- WO-A-97/17084
- WO-A-98/13378
- A. MABONDZO ET AL.: "ANTIBODY-DEPENDENT CELLULAR CYTOTOXICITY AND NEUTRALIZATION OF HUMAN IMMUNODEFICIENCY VIRUS TYPE 1 BY HIGH AFFINITY CROSS-LINKING OF gp41 TO HUMAN MACROPHAGE Fc IgG RECEPTOR USING BISPECIFIC ANTIBODY" JOURNAL OF GENERAL VIROLOGY, vol. 75, 1994, pages 1451-1456, XP002132667

## Description

The invention relates to new molecular complexes presenting high affinity binding with respect to monocyte derived cells and their uses in therapy.

Blood monocytes in physiological conditions leave the blood stream flow to reach tissues where they differentiate into resident macrophages (for example: lung macrophages, kupffer cells in liver, skin macrophages, osteoclasts in bone, microglial cells in brain ...), or into professional antigen presenting cells (for example : dendritic cells in peripheral tissues or lymphnodes, Langerhans cells in skin ...).

Differentiation of blood monocytes can also be achieved *ex vivo* under defined culture conditions (see applications WO94/26875, WO96/22781, WO97/44441, WO99/13054); however, the macrophages or the dendritic cells obtained in culture do not achieve tissue specificity similar to the one obtained *in vivo*).

Furthermore, the induction of an immune response has been documented when the antigens are known. However, regarding the induction of an immune response towards unknown antigens (particularly tumor antigens), a targeting of these antigens to specific receptors of the antigen presenting cells is required ; this is an objective of the present invention.

One of the aims of the invention is to provide monocyte derived cells which have acquired a tissue specificity.

Another aim of the invention is to provide an *ex vivo* method for stimulating cellular and/or humoral immune responses against unknown components of a tumor tissue extract.

Another aim of the invention is to provide *in vivo* specific cellular and/or humoral immune responses against unknown component of tumor tissue extract.

All these aims are achieved through the invention, which gives access to new molecular complexes having high affinity with tissue extracts on the one hand, and high affinity with monocyte derived cells on the other hand.

More precisely, the invention relates to a molecular complex between a tissue extract containing at least one known component and unknown components and a molecular vector comprising a particle bearing polypeptides and/or sugars, said molecular vector being able to recognize :
- said known component of said tissue extract, and
- a phagocytic receptor of monocyte derived cells,
with the proviso that polypeptides are different from antibodies.

The expression *"known component"* means identified tissue antigens, polypeptides or oligosaccharides or an hapten expressed or transfected on the cell membrane of tissues or tumors.

The expression *"unknown component"* means "complex mixture of proteins and saccharides present in cellular extracts of tumors or tissues (lysates, apoptotic extracts,...)

The expression "*molecular vector"* corresponds to a carrier of molecular structure.

The expression *"recognize said known component of said tissue extract*" means that it presents a high affinity and/or avidity (> 10⁻⁶ M) for said component.

The expression *"recognize a phagocytic receptor of monocyte derived cells"* means that it is a ligand for such receptor.

The expression "*polypeptides are different from antibodies"* means that they are not monoclonal or polyclonal antibodies with Fc and Fab parts.

A phagocytic receptor of monocyte derived cells is a receptor such that, when interacting with a ligand, in this case, the molecular complex, it initiates uptake of said ligand.

The phagocytic status means that the monocyte derived cells have gained, after a few days of culture, for instance, about 4 to about 10 days, a high phagocytic activity. (This phagocytic activity can be visualized and quantified by measuring, for instance under the microscope, the uptake of yeast particles).

The expression *"monocyte derived cells (or MDCs)*" designates macrophages or dendritic cells derived from blood monocytes.

According to an advantageous embodiment, the invention relates to a molecular complex wherein the molecular vector comprises a particle bearing polypeptides and/or sugars such that:
- at least one of the said polypeptides and/or sugars recognizes said known surface component of the tissue extract,
- at least one of the said sugars and/or polypeptides recognizes phagocytic receptors of monocyte derived cells such as receptors for mannose or for oligosaccharides or Fc receptors of monocyte derived cells.

There are thus four different possibilities :
1) at least one of the said polypeptides of the particle can recognize a known component of the tissue extract and at least one of the said polypeptides of the particle can recognize a phagocytic receptor of monocyte derived cells,
2) at least one of the said polypeptides of the particle can recognize a known component of the tissue extract and at least one of the said sugars of the particle can recognize a phagocytic receptor of monocyte derived cells,
3) at least one of the said sugars can recognize a known component of the tissue extract and at least one of the said sugars of the particle can recognize a phagocytic receptor of monocyte derived cells,
4) at least one of the said sugars can recognize a known component of the tissue extract and at least one of the said polypeptides of the particle can recognize a phagocytic receptor of monocyte derived cells.

The nature of the bond between the sugar and the known component is formed of hydrogen, Vanderwaals, hydrophobic interactions and salt bridges.

The nature of the bond between the sugar and the monocyte derived cells is mainly hydrogen, Vanderwaals, hydrophobic interactions and salt bridges.

The nature of the bond between the polypeptides and the known component is mainly hydrogen, Vanderwaals, hydrophobic interactions and salt bridges.

The nature of the bond between the polypeptide and monocyte derived cells is mainly hydrogen, Vanderwaals, hydrophobic interactions and salt bridges.

In an advantageous embodiment of the molecular complex of the invention, the molecular vector comprises or is a particle of about 0,1 to about 2 µm of biocompatible polymer comprising :
- surface polypeptides and/or sugars, preferably covalently linked to the surface of said particle, with said surface polypeptides and/or sugars recognizing said known component of the tissue extract, and
- mannosylated residues recognizing the mannose or oligosaccharide receptors of monocyte derived cells.

According to an advantageous embodiment, in the molecular complex of the invention, the tissue extract comprises macroscopic fragments or killed or irradiated or haptenised human or animal tumor cells such as lysates or apoptotic bodies, or killed pathogens, such as viruses or bacteria.

According to an advantageous embodiment, in the molecular complex of the invention, the polypeptide of the particle recognises one known epitope of the tissue extract chosen among known tumor antigens such as (tumor peptide antigen) MelanA/MART-1, MAGE, BAGE, GAGE families, MUC, EGF-R, ERB-2, PSA, PSMA, HSP70, CEA, P53, RAS, Tyrosinase, gp100.

According to another advantageous embodiment, in the molecular complex of the invention, the tissue extract comprises normal tissue parts such as tissue membranes, tissue factors, tissue proteins, macroscopic fragments of tissue such as lysates or apoptopic bodies, said tissue being originating from any part of human or animal body or cellular extracts thereof, in particular from thymus, lung, pancreas, cartilage, endothelium, neuromuscular junctions, prostate, sexual organs, bladder, muscles, peripheral nerves, CNS extracts, spleen, liver, bone, heart, skin cells.

In the molecular complex of the invention, the polypeptide and/or sugars of said particle form(s) high affinity binding with any component of said tissue extract.

In the molecular complex of the invention, the polypeptide and/or sugars of the particle form(s) high affinity binding with a phagocytic receptor of a monocyte derived cell.

The expression *"high affinity binding*" means that the affinity constant Ka is equal to or higher than 10⁶ M or the equilibrium dissociation constant K_{D} is equal to or lower than 10⁻⁶ M.

According to an advantageous embodiment, the monocyte derived cells recognized by the molecular complex of the invention are macrophages, dendritic cells, or antigen presenting cells.

The invention also relates to monocyte derived cells such as prepared according to a process comprising the step of contacting monocyte derived cells with a molecular complex according to the invention.

The invention also relates to monocyte derived cells such as prepared according to a process comprising contacting monocyte derived cells with a molecular complex according to the invention, under conditions enabling phagocytosis of said molecular complex by said monocyte derived cells, intracellular degradation and processing of the known and unknown components of the tumor tissue extract and the presentation of said known and unknown components on the peripheral membrane of the monocyte derived cells together with MHC 1 and MHC II molecules.

The monocyte derived cells are immature dendritic cells for the phagocytosis, which then mature for the induction of immune response.

The invention also relates to monocyte derived cells such as prepared according to a process comprising contacting monocyte derived cells with a molecular complex as described above, under conditions enabling phagocytosis of such molecular complex by the monocyte derived cells.

The monocyte derived cells are non-activated macrophages (4/8 days of culture).

The invention also relates to an ex vivo method for stimulating cellular and/or humoral immune responses against unknown components of a tumor tissue extract comprising contacting monocyte derived cells with a molecular complex according to the invention, under conditions enabling phagocytosis of said molecular complex by monocyte derived cells, intracellular degradation and processing of the known and of unknown components of the tumor tissue extract and the presentation of said known and unknown components on the peripheral membrane of the monocyte derived cells, together with MHC I and II molecules.

The invention also relates to the use of a molecular complex for the manufacture of a medicament for inducing in vivo specific cellular and/or humoral immune responses against unknown components of tumor tissue extract comprising injections of a molecular complex according to the invention, for instance by intramuscular, subcutaneous, local or intravenous route.

According to an advantageous embodiment, the invention relates to the use of monocyte derived cells presenting known and unknown components of said tumor tissue extract, together with MHC I and II molecules, as defined above, and of molecular complexes as described above in the form of sequential and/or simultaneous injections; for the manufacture of a medicament for inducing in vivo specific cellular and/or humoral responses against unknown components of said tumor tissue extract.

The invention also relates to a method for conditioning *ex vivo* monocytes derived cells, and preferentially macrophages, for them to acquire tissue specificity, comprising contacting monocyte derived cells with a molecular complex according to the invention, under conditions enabling phagocytosis of said molecular complex by the monocyte derived cells.

The expression *"conditioning ex vivo human monocyte derived cells"* means that after phagocytosis of specific tissue extracts, the MDCs acquire characteristics of the corresponding tissue macrophages.

The expression *"acquire tissue specificity*" means that when the MDCs are injected *in vivo*, they will (concentrate) accumulate preferentially in the corresponding tissue.

The invention also relates to the use of monocyte derived cells and of a molecular complex according to the invention in the form of simultaneous and/or sequential injections, under conditions enabling phagocytosis, or of monocyte derived cells which have previously phagocytosed a molecular complex according to the invention, in the form of injections, for the manufacture of a medicament for testing diseases involving accumulation of conditioned monocyte derived cells as described above in specific tissue to induce tissue repair and/or regeneration.

The expression "*accumulation of conditioned monocyte derived cells*" in a tissue means that, after systemic injection, at least 10% of the cells injected accumulate in the tissue within 24 h.

In the invention, the monocyte derived cells which are advantageously involved are human monocyte derived cells.

By way of example, the diseases which can be treated by the method of the invention are tissue/organ destruction or degenerative diseases, when tissue repair is required (skin, bone, nerve, neuromuscular regeneration).

The invention also relates to pharmaceutical compositions comprising, as active substance, monocyte derived cells which have been contacted with a molecular complex according to the invention, under conditions enabling phagocytosis of said molecular complex by monocyte derived cells, intracellular degradation and processing of the known and of unknown components of the tumor tissue extract and the presentation of said known and unknown components on the peripheral membrane of the monocyte derived cells, together with MHC I and II molecules.

The invention also relates to pharmaceutical compositions comprising, as active substance, monocyte derived cells, and preferentially macrophages, which have been contacted with a molecular complex according to the invention, under conditions enabling phargocytosis of said molecular complex by the monocyte derived cells.

### EXAMPLE 1 : Application to a human melanoma tumor:

Apoptotic bodies are generated from a human melanoma cell line M17 by UV irradiation. They are added in basic medium to microparticles of 0.2 to 2 µm with covalently linked annexin V polypeptides and mannosyl residues. Annexin V presents a high affinity for phosphatidyl serine residues expressed on apoptotic bodies.

The microparticles contain a magnetic core and the molecular complexes (tumor apoptotic bodies - microparticles) are isolated on magnets. A working bank of molecular melanoma complexes is constituted and kept frozen.

Patients with metastatic melanoma are injected into 4 subcutaneous sites, one intradermal site plus one intravenous site with the defrost preparation.

The injections are repeated after 2 weeks and again one month later. Interaction with dendritic cells is occurring locally in the patient.

The induction of a specific immune response against the melanoma tumor is documented by humoral and cellular T responses against the known MAGE and MelanA/MART antigens expressed by the M17 cell line. The global antitumoral effect is shown by shrinkage (> 50%) of subcutaneous metastases, this response requires immune activation against multiple melanoma tumor antigens or than the targeted antigen.

### EXAMPLE 2 : Application to tissue repair in a murine model:

Microparticles of 0.2 to 2 µm size presenting at their surface mannosyl residues are added to a suspension of killed murine hepatocytes, and molecular complexes are formed.

Macrophages are obtained by differentiation of murine bone marrow cells in culture and labelled with indium or an emittor of positons (example: Fluor 18).

These macrophages are grown for 16 h in the presence (a) or the absence (b) of molecular complexes.

Two millions of these macrophages are injected intravenously to the mice. After 2 hours, the biodistribution of the macrophages in the animal tissues is measured by gamma counting or PET-scan (SMV International). In case (a), 90% of the macrophages injected are in the liver while in case (b), only 20% of the macrophages are in liver. This indicates that the macrophages grown in the presence of the molecular complexes have gained a liver tissue specificity. If necrosis of the liver is previously induced, a fast regeneration is seen a few days after macrophage injection.

## Claims

1. Molecular complex between a tissue extract containing at least one known component and unknown components and a molecular vector comprising a particle bearing sugars and/or polypeptides, said molecular vector being able to recognize :
- said known component of said tissue extract, and
- a phagocytic receptor of monocyte derived cells,
with the proviso that said polypeptides are different from antibodies.

2. Molecular complex according to claim 1, wherein the molecular vector comprises a particle bearing polypeptides and/or sugars such that:
- at least one of the said polypeptides and/or sugars recognizes said known surface component of the tissue extract,
- at least one of the said sugars and/or polypeptides recognizes phagocytic receptors of monocyte derived cells such as receptors for mannose or for oligosaccharides or Fc receptors of monocyte derived cells.

3. Molecular complex according to claim 2, wherein the molecular vector comprises or is a particle of about 0,1 to about 2 µm of biocompatible polymer comprising
- surface polypeptides and/or sugars, preferably covalently linked to the surface of said particle, with said surface polypeptides and/or sugars recognizing said known component of the tissue extract, and
- mannosylated residues recognizing the mannose or oligosaccharide receptors of monocyte derived cells.

4. Molecular complex according to anyone of claims 1 to 3, wherein the tissue extract comprises macroscopic fragments or killed or irradiated or haptenized human or animal tumor cells such as lysates or apoptotic bodies, or killed pathogens, such as viruses or bacteria.

5. Molecular complex according to claim 4, wherein the polypeptide of the particle recognises one known epitope of the tissue extract chosen among known tumor antigens such as (tumor peptide antigen) MelanA/MART-1, MAGE, BAGE, GAGE families ; MUC, EGF-R, ERB-2, PSA, PSMA, HSP70, CEA, P53, RAS, Tyrosinase, gp100.

6. Molecular complex according to anyone of claims 1 to 3, wherein the tissue extract comprises normal tissue parts such as tissue membranes, tissue factors, tissue proteins, macroscopic fragments of tissue such as lysates or apoptopic bodies, said tissue being originating from any part of human or animal body or cellular extracts thereof, in particular from thymus, lung, pancreas, cartilage, endothelium, neuromuscular junctions, prostate, sexual organs, bladder, muscles, peripheral nerves, CNS extracts, spleen, liver, bone, heart, skin cells.

7. Molecular complex according to any one of claims 1 to 6, wherein the polypeptide and/or sugars of said particle forms high affinity binding with any component of said tissue extract.

8. Molecular complex according to anyone of claims 1 to 7, wherein the monocyte derived cells recognized by said molecular complex are macrophages, dendritic cells, or antigen presenting cells.

9. Monocyte derived cells such as prepared according to a process comprising the step of contacting monocyte derived cells with a molecular complex according to anyone of claims 1 to 8.

10. Monocyte derived cells such as prepared according to a process comprising contacting monocyte derived cells with a molecular complex according to anyone of claims 1 to 5, 7 and 8, under conditions enabling phagocytosis of said molecular complex by said monocyte derived cells, intracellular degradation and processing of the known and unknown components of the tumor tissue extract and the presentation of said known and unknown components on the peripheral membrane of the monocyte derived cells together with MHC I and MHC II molecules.

11. Monocyte derived cells such as prepared according to a process comprising contacting monocyte derived cells with a molecular complex according to any one of claims 1 to 3, 6, 7 and 8, under conditions enabling phagocytosis of such molecular complex by the monocyte derived cells.

12. *Ex vivo* method for stimulating cellular and/or humoral immune responses against unknown components of a tumor tissue extract comprising contacting monocyte derived cells with a molecular complex according to anyone of claims 1 to 5, 7 and 8, under conditions enabling phagocytosis of said molecular complex by monocyte derived cells, intracellular degradation and processing of the known and of unknown components of the tumor tissue extract and the presentation of said known and unknown components on the peripheral membrane of the monocyte derived cells, together with MHC I and II molecules.

13. Use of a molecular complex according to anyone of claims 1 to 5, 7 and 8, for the manufacture of a medicament for inducing *in vivo* specific cellular and/or humoral immune responses against unknown components of tumor tissue extract.

14. Use of monocyte derived cells presenting known and unknown components of a tumor tissue extract, together with MHC I and II molecules, as defined in claim 12, and of molecular complexes according to anyone of claims 1 to 5, for the manufacture of a medicament for inducing *in vivo* specific cellular and/or humoral responses against unknown components of said tumor tissue extract.

15. Method for conditioning *ex vivo* human monocytes derived cells, and preferentially macrophages, for them to acquire tissue specificity, comprising contacting monocyte derived cells with a molecular complex according to anyone of claims 1 to 8, under conditions enabling phagocytosis of such molecular complex by the monocyte derived cells.

16. Use of:
- monocyte derived cells and of a molecular complex according to anyone of claims 1 to 8, under conditions enabling phagocytosis of such molecular complex by the monocyte derived cells,
- or of monocyte derived cells which have previously phagocytosed a molecular complex according to anyone of claims 1 to 8,
for the manufacture of a medicament for the treatment of diseases involving accumulation of conditioned monocyte derived cells according to claim 15 in specific tissue to induce tissue repair and/or regeneration.

## Revendications

1. Complexe moléculaire entre un extrait tissulaire contenant au moins un composant connu et des composants inconnus et entre un vecteur moléculaire comprenant une particule portant des sucres et/ou des polypeptides, ledit vecteur moléculaire étant capable de reconnaître :
- ledit composant connu dudit extrait tissulaire, et
- un récepteur phagocytaire de cellules dérivées de monocytes,
sous réserve que lesdits polypeptides soient différents d'anticorps.

2. Complexe moléculaire selon la revendication 1, dans lequel le vecteur moléculaire comprend une particule portant des polypeptides et/ou des sucres telle que :
- au moins un desdits polypeptides et/ou sucres reconnaît ledit composant de surface connu de l'extrait tissulaire,
- au moins un desdits sucres et/ou polypeptides reconnaît les récepteurs phagocytaires des cellules dérivées de monocytes tels que les récepteurs du mannose ou des oligosaccharides ou les récepteurs Fc des cellules dérivées de monocytes.

3. Complexe moléculaire selon la revendication 2, dans lequel le vecteur moléculaire comprend ou est une particule d'environ 0,1 à environ 2 µm de polymère biocompatible comprenant :
- des polypeptides et/ou des sucres de surface, de préférence liés de façon covalente à la surface de ladite particule, lesdits polypeptides et/ou sucres de surface reconnaissant ledit composant connu de l'extrait tissulaire, et
- des résidus mannosylés reconnaissant les récepteurs du mannose ou des oligosaccharides des cellules dérivées de monocytes.

4. Complexe moléculaire selon l'une quelconque des revendications 1 à 3, dans lequel l'extrait tissulaire comprend des fragments macroscopiques ou des cellules tumorales, animales ou humaines, tuées ou irradiées ou hapténisées, telles que des lysats ou des corps apoptotiques, ou des pathogènes tués tels que des virus ou des bactéries.

5. Complexe moléculaire selon la revendication 4, dans lequel le polypeptide de la particule reconnaît un épitope connu de l'extrait tissulaire choisi parmi les antigènes tumoraux connus tels que (antigène peptidique tumoral) MelanA/MART-1, les familles MAGE, BAGE, GAGE ; MUC, EGF-R, ERB-2, PSA, PSMA, HSP70, CEA, P53, RAS, Tyrosinase, gp100.

6. Complexe moléculaire selon l'une quelconque des revendications 1 à 3, dans lequel l'extrait tissulaire comprend des parties de tissu normal telles que des membranes tissulaires, des facteurs tissulaires, des protéines tissulaires, des fragments macroscopiques de tissu tels que des lysats ou des corps apoptotiques, ledit tissu provenant de toute partie du corps humain ou animal ou d'extraits cellulaires de celui-ci, notamment de cellules du thymus, du poumon, du pancréas, du cartilage, de l'endothélium, des jonctions neuromusculaires, de la prostate, des organes sexuelles, de la vessie, des muscles, des nerfs périphériques, des extraits du SNC, de la rate, du foie, des os, du coeur et de la peau.

7. Complexe moléculaire selon l'une quelconque des revendications 1 à 6, dans lequel les polypeptides et/ou les sucres de ladite particule forment une liaison de forte affinité avec tout composant dudit extrait tissulaire.

8. Complexe moléculaire selon l'une quelconque des revendications 1 à 7, dans lequel les cellules dérivées de monocytes reconnues par ledit complexe moléculaire sont des macrophages, des cellules dendritiques ou des cellules présentatrices d'antigènes.

9. Cellules dérivées de monocytes telles que préparées selon un procédé comprenant l'étape de mise en contact de cellules dérivées de monocytes avec un complexe moléculaire selon l'une quelconque des revendications 1 à 8.

10. Cellules dérivées de monocytes telles que préparées selon un procédé comprenant la mise en contact de cellules dérivées de monocytes avec un complexe moléculaire selon l'une quelconque des revendications 1 à 5, 7 et 8, dans des conditions permettant la phagocytose dudit complexe moléculaire par lesdites cellules dérivées de monocytes, la dégradation intracellulaire et le traitement des composants connus et inconnus de l'extrait tissulaire tumoral et la présentation desdits composants connus et inconnus sur la membrane périphérique des cellules dérivées de monocytes avec les molécules CMH I et CMH II.

11. Cellules dérivées de monocytes telles que préparées selon un procédé comprenant la mise en contact de cellules dérivées de monocytes avec un complexe moléculaire selon l'une quelconque des revendications 1 à 3, 6, 7 et 8, dans des conditions permettant la phagocytose d'un tel complexe moléculaire par les cellules dérivées de monocytes.

12. Procédé *ex vivo* de stimulation des réponses immunitaires cellulaires et/ou humorales vis-à-vis de composants inconnus d'un extrait tissulaire tumoral comprenant la mise en contact de cellules dérivées de monocytes avec un complexe moléculaire selon l'une quelconque des revendications 1 à 5, 7 et 8, dans des conditions permettant la phagocytose dudit complexe moléculaire par les cellules dérivées de monocytes, la dégradation intracellulaire et le traitement des composants connus et inconnus de l'extrait tissulaire tumoral et la présentation desdits composants connus et inconnus sur la membrane périphérique des cellules dérivées de monocytes, avec les molécules CMH I et II.

13. Utilisation d'un complexe moléculaire selon l'une quelconque des revendications 1 à 5, 7 et 8 pour la fabrication d'un médicament pour induire *in vivo* des réponses immunitaires cellulaires et/ou humorales spécifiques vis-à-vis de composants inconnus de l'extrait tissulaire tumoral.

14. Utilisation de cellules dérivées de monocytes présentant les composants connus et inconnus d'un extrait tissulaire tumoral, avec des molécules CMH I et II, comme défini dans la revendication 12, et de complexes moléculaires selon l'une quelconque des revendications 1 à 5, pour la fabrication d'un médicament pour induire *in vivo* des réponses immunitaires cellulaires et/ou humorales spécifiques vis-à-vis de composants inconnus dudit extrait tissulaire tumoral.

15. Procédé pour le conditionnement *ex vivo* de cellules humaines dérivées de monocytes, et de préférence des macrophages pour leur faire acquérir une spécificité tissulaire, comprenant la mise en contact de cellules dérivées de monocytes avec un complexe moléculaire selon l'une quelconque des revendications 1 à 8, dans des conditions permettant la phagocytose d'un tel complexe moléculaire par les cellules dérivées de monocytes.

16. Utilisation :
- de cellules dérivées de monocytes et d'un complexe moléculaire selon l'une quelconque des revendications 1 à 8, dans des conditions permettant la phagocytose d'un tel complexe moléculaire par les cellules dérivées de monocytes, ou
- de cellules dérivées de monocytes qui ont phagocyté au préalable un complexe moléculaire selon l'une quelconque des revendications 1 à 8,
pour la fabrication d'un médicament destiné au traitement de maladies impliquant l'accumulation de cellules dérivées de monocytes conditionnées selon la revendication 15 dans un tissu spécifique afin d'induire une réparation et/ou une régénération tissulaire.

## Patentansprüche

1. Molekülkomplex zwischen einem Gewebeextrakt, welcher wenigstens einen bekannten Bestandteil und unbekannte Bestandteile enthält, und einen Molekülvektor mit einer Partikel, welcher Zucker und/oder Polypeptide trägt, wobei der Molekülvektor erkennen kann:
- den bekannten Bestandteil des Gewebeextrakts, und
- einen phagozytischen Rezeptor von Zellen, die von Monozyten abgeleitet sind,
unter der Bedingung, dass die Polypeptide unterschiedlich zu Antikörpern sind.

2. Molekülkomplex nach Anspruch 1, wobei der Molekülvektor einen Partikel umfasst, welcher Polypeptide und/oder Zucker trägt, in der Art, dass:
- wenigstens eines der Polypeptide und/oder Zucker den bekannten Oberflächenbestandteil des Gewebeextrakts erkennt,
- wenigstens einer der Zucker und/oder Polypeptide die phagozytische Rezeptoren von Zellen erkennt, die von Monozyten abgeleitet sind, wie etwa Rezeptoren für Mannose oder für Oligosaccharide oder Fc-Rezeptoren von Zellen, die von Monozyten abgeleitet sind.

3. Molekülkomplex nach Anspruch 2, wobei der Molekülvektor einen Partikel von etwa 0,1 bis etwa 2 µm eines bioverträglichen Polymers umfasst oder ist, mit
- Oberflächenpolypeptiden und/oder Zuckern, bevorzugt kovalent gebunden an die Oberfläche des Partikels mit den Oberflächenpolypeptiden und/oder Zuckern, welche den bekannten Bestandteil des Gewebeextrakts erkennen, und
- mannosylierte Reste, welche die Mannose oder Oligosaccharid-Rezeptoren von Zellen erkennen, die von Monozyten abgeleitet sind.

4. Molekülkomplex nach einem der Ansprüche 1 bis 3, wobei der Gewebeextrakt makroskopische Fragmente oder getötete oder bestrahlte oder haptenisierte menschliche oder tierische Tumorzellen, wie etwa Lysate oder apoptotische Körper oder abgetötete Pathogene, wie etwa Viren oder Bakterien, umfasst.

5. Molekülkomplex nach Anspruch 4, wobei die Polypeptide des Partikels ein bekanntes Epitop des Gewebeextrakts erkennen, ausgewählt aus bekannten Tumorantigenen wie etwa (Tumorpeptidantigen) MelanA/MART-1-, MAGE-, BAGE-, GAGE-Familien; MUC, EGF-R, ERB-2, PSA, PSMA, HSP70, CEA, P53, RAS, Tyrosinase, gp100.

6. Molekülkomplex nach einem der Ansprüche 1 bis 3, wobei der Gewebeextrakt normale Gewebeteile umfasst, wie etwa Gewebemembranen, Gewebefaktoren, Gewebeproteine, makroskopische Fragmente von Gewebe, wie etwa Lysate oder apoptotische Körper, wobei das Gewebe von einem Teil des menschlichen oder tierischen Körpers oder Zellextrakten davon stammt, insbesondere vom Thymus, der Lunge, des Pankreas, Knorpel, Endothel, neuromuskulären Verbindungen, der Prostata, den Sexualorganen, der Blase, den Muskeln, den peripheren Nerven, CNS-Extrakten, der Milz, der Leber, den Knochen, dem Herz, Hautzellen.

7. Molekülkomplex nach einem der Ansprüche 1 bis 6, wobei das Polypeptid und/oder die Zucker des Partikels eine hochaffine Bindung mit einem Bestandteil des Gewebeextrakts ausbilden.

8. Molekülkomplex nach einem der Ansprüche 1 bis 7, wobei die durch den Molekülkomplex erkannten Zellen, die von Monozyten abgeleitet sind, Makrophagen, dendritische Zellen oder Antigen-präsentierende Zellen sind.

9. Zellen, die von Monozyten abgeleitet sind, gemäß einem Verfahren hergestellt, welches den Schritt des in Kontaktbringens von Zellen, die von Monozyten abgeleitet sind, mit einem Molekülkomplex nach einem der Ansprüche 1 bis 8 umfasst.

10. Zellen, die von Monozyten abgeleitet sind, gemäß einem Verfahren hergestellt, welches das in Kontaktbringen von Zellen, die von Monozyten abgeleitet sind, mit einem Molekülkomplex nach einem der Ansprüche 1 bis 5, 7 und 8 umfasst, unter Bedingungen, welche die Phagozytose des Molekülkomplexes durch die Zellen, die von Monozyten abgeleitet sind, den intrazellulären Abbau und die Prozessierung der bekannten und unbekannten Bestandteile des Tumorgewebeextrakts und die Präsentation der bekannten und unbekannten Bestandteile auf der peripheren Membran der Zellen, die von Monozyten abgeleitet sind, zusammen mit MHC I- und MHC II-Molekülen umfassen.

11. Zellen, die von Monozyten abgeleitet sind, gemäß einem Verfahren hergestellt, mit in Kontaktbringen von Zellen, die von Monozyten abgeleitet sind mit einem molekularen Komplex nach einem der Ansprüche 1 bis 3, 6, 7 und 8, unter Bedingungen, welche die Phagozytose des Molekülkomplexes durch die Zellen ermöglicht, die von Monozyten abgeleitet sind.

12. *Ex vivo*-Verfahren zur Stimulierung zellulärer und/oder humoraler Immunantworten gegen unbekannte Bestandteile eines Tumorgewebeextrakts, mit in Kontaktbringen von Zellen, die von Monozyten abgeleitet sind, mit einem Molekülkomplex nach einem der Ansprüche 1 bis 5, 7 und 8, unter Bedingungen, welche die Phagozytose des Molekülkomplexes durch Zellen, die von Monozyten abgeleitet sind, den intrazellulären Abbau und die Prozessierung der bekannten und unbekannten Bestandteile des Tumorgewebeextrakts und die Präsentation der bekannten und unbekannten Bestandteile auf der peripheren Membran der Zellen, die von Monozyten abgeleitet sind, zusammen mit MHC I- und MHC II-Molekülen umfassen.

13. Verwendung eines molekularen Komplexes nach einem der Ansprüche 1 bis 5, 7 und 8, für die Herstellung eines Arzneimittels zur *in vivo*-Induktion spezifischer zellulärer und/oder humoraler Immunantworten gegen unbekannte Bestandteile von Tumorgewebeextrakt.

14. Verwendung von Zellen, die von Monozyten abgeleitet sind, welche bekannte und unbekannte Bestandteile des Tumorgewebeextrakts zusammen mit MHC I- und MHC II-Molekülen, nach Anspruch 12, und Molekülkomplexen nach einem der Ansprüche 1 bis 5 präsentieren, für die Herstellung eines Arzneimittels zur *in vivo*-Induktion spezifischer zellulärer und/oder humoraler Antworten gegen unbekannte Bestandteile eines Tumorgewebeextrakts.

15. Verfahren zur *ex vivo*-Konditionierung menschlicher Zellen, die von Monozyten abgeleitet sind, und bevorzugt Makrophagen, damit sie Gewebespezifität erwerben, mit in Kontaktbringen von Zellen, die von Monozyten abgeleitet sind, mit einem Molekülkomplex nach einem der Ansprüche 1 bis 8, unter Bedingungen, welche die Phagozytose des Molekülkomplexes durch Zellen ermöglichen, die von Monozyten abgeleitet sind.

16. Verwendung
- von Zellen, die von Monozyten abgeleitet sind, und eines Molekülkomplexes nach einem der Ansprüche 1 bis 8 unter Bedingungen, welche Phagozytose eines solchen Molekülkomplexes durch Zellen, die von Monozyten abgeleitet sind, ermöglichen,
- oder von Zellen, die von Monozyten abgeleitet sind, welche vorher einen Molekülkomplex nach einem der Ansprüche 1 bis 8 phagozytiert haben,
für die Herstellung eines Arzneimittels zur Behandlung von Krankheiten, welches die Anreicherung von konditionierten Zellen nach Anspruch 15, die von Monozyten abgeleitet sind, in spezifischem Gewebe umfasst, um Gewebereparatur und/oder - regeneration zu induzieren.
